# EUROPÄISCHE PATENTSCHRIFT

(11) **EP 1 875 233 B1**
(45) Veröffentlichungstag und Bekanntmachung des Hinweises auf die Patenterteilung: **09.09.2009**
(21) Anmeldenummer: 06724731.2
(22) Anmeldetag: 25.04.2006
(51) Int. Cl.: G01N 33/497, G01N 33/487, A61B 5/097

(54) **VERFAHREN UND VORRICHTUNG ZUR GEWINNUNG UND ANALYSE VON ATEMKONDENSATEN**
METHOD AND DEVICE FOR RECOVERING AND ANALYZING RESPIRATORY CONDENSATES
PROCEDE ET DISPOSITIF POUR RECUEILLIR ET ANALYSER DES CONDENSATS DE RESPIRATION

(30) Priorität: 25.04.2005 DE 102005020102
(43) Veröffentlichungstag der Anmeldung: 09.01.2008
(73) Patentinhaber: Universität Potsdam, 14469 Potsdam (DE)
(72) Erfinder: SCHÖNFUSS, Dirk, 12542 Berlin (DE); SZEPONIK, Jan, 13187 Berlin (DE); GALL, Ingrid, 16341 Zepernick (DE)
(74) Vertreter: Rasch, Dorit
(86) Internationale Anmeldenummer: PCT/EP2006/004227
(87) Internationale Veröffentlichungsnummer: WO 2006/114335

(56) Entgegenhaltungen:
- EP-A- 0 634 488
- DE-A1- 10 228 088
- DE-A1- 19 528 158
- DE-A1- 19 951 204

## Beschreibung

Die Erfindung betrifft ein Verfahren zur Analyse von Inhaltsstoffen der Ausatemluft unter Verwendung von elektrochemischen Sensoren und/oder Biosensoren. Die Bestimmung erfolgt durch Messung und Auswertung von Signalen, welche ein oder mehrere der Sensoren bei Kontakt mit diesen Substanzen oder mit aus diesen Substanzen entstehenden Reaktionsprodukten erzeugen. Diese zu untersuchenden Substanzen werden bei der Kondensation des im Atemgas enthaltenen Wassers an einer Kondensationsfläche ebenfalls kondensiert. Das entstandene Atemkondensat wird den Sensoren direkt zugeführt. Die Kondensationsfläche ist dabei funktionalisiert bzw. aktiviert, so dass eine Wechselwirkung bzw. eine chemische Reaktion während des Kondensationsprozesses mit dem Kondensat und/oder den zu analysierenden Substanzen erfolgt, wodurch das an den Sensoren erzeugte Signal beeinflusst wird. Die Erfindung betrifft auch eine Vorrichtung zur Gewinnung von Atemkondensaten. Diese umfasst auf einem Grundelement (einer Halterung bzw. einem Träger) mindestens eine Sensoreinheit, die neben den Sensoren eine entsprechend funktionalisierte oder aktivierte Kondensationsfläche aufweist. Des Weiteren umfasst sie mindestens ein Peltier-Element und eine Wärmeleitbrücke. In einer bevorzugten Ausführungsvariante der Erfindung kann das Grundelement mit einer Kopfhalterung verbunden sein und so direkt im Atemstrom unmittelbar vor dem Mund des Trägers positioniert und gegebenenfalls mit einem tragbaren Stromversorgungs- und elektronischen Auswertesystem verbunden werden.

Verschiedene Verfahren und Vorrichtungen zur Gewinnung von Atemkondensat und der Konzentrationsbestimmung von Substanzen im Atemkondensat sind bekannt. In DE 199 51 204 C2 wird ein Verfahren zur Analyse der Inhaltsstoffe der Ausatemluft beschrieben, bei dem eine Kühlung der Ausatemluft bis zur Kondensation ihrer aerosol- und dampfförmigen Bestandteile bis zum Erreichen einer vorgegebenen Probenmenge erfolgt. Die vorgegebene Probenmenge wird dabei durch eine vollständige Füllung oder Sättigung einer Speicherschicht oder eines Filters festgelegt. Die Bestimmung der im Kondensat enthaltenen Substanzen erfolgt an einem oder mehreren elektrochemischen Sensoren, welche der Speicherschicht oder dem Filter nachgeordnet sind, wobei das Atemkondensat durch den Filter oder die Speicherschicht auf die Sensoren diffundiert. Der Nachteil dieser und ähnlicher Lösungen besteht darin, dass das Atemkondensat zunächst in dem Filter oder der Speicherschicht gefangen ist und somit ein großer Teil der Probe dem analytischen Prozess vorenthalten bleibt und kurzlebige Substanzen (z.B. Radikale) teilweise oder bereits gar nicht mehr vorliegen, da erst nach vollständiger Füllung des Filters oder der Speicherschicht mit Atemkondensat dieses auf die nachgeordneten Sensoren durch Diffusion gelangt. Ebenfalls nachteilig sind die Gefahren von Infektionen und Verunreinigungen der Probe und die deshalb nach jeder Analyse erforderliche Desinfektion. In DE 195 28 158 A1 wird eine Vorrichtung zur Gewinnung der Inhaltsstoffe der Ausatemluft beschrieben, bei der eine Kondensation der Inhaltsstoffe auf einer nichtadsorptiven Folie, welche auf einem Peltierelement fixiert ist, erfolgt. DE 101 375 65 A1 beschreibt die Bestimmung von Parametern des Atemkondensates mit Hilfe einer geschlossenen Kassette, in der sowohl die Sensoren selbst als auch Puffer-, Kalibrier- und eventuell Verdünnungslösungen zur Änderung der Leitfähigkeit der Probe in Vorratsbehältern oder auswechselbaren Kartuschen untergebracht sind. Die Ausbringung der Lösungen aus den Vorratsbehältern auf die Sensoren zum Zwecke der Messung, Sensorkonditionierung oder Kalibrierung der Sensoren erfolgt mit Hilfe einer speziellen Apparatur, welche auf die Vorratsbehälter einwirkt und nicht Bestandteil der Kassette ist. Die Probelösung, das Atemkondensat, wird vor der Messung in die Kassette aus einem Probengefäß oder einem Probensammelsystem gesaugt oder eingespritzt und vor dem Ausbringen auf die Sensoren einer Vermischung mit innerhalb oder außerhalb der Kassette untergebrachten Substanzen oder Lösungen zum Zwecke der Verdünnung und/oder Ionenkonzentrations- und/oder Leitfähigkeitsänderung unterzogen. Nach der Messung wird die Kassette entsorgt. Nachteilig ist der große gerätetechnische Aufwand für die Gewinnung einer ausreichenden Menge an Probe, die mit einer gesonderten Apparatur erfolgt, wonach die Probe gegebenenfalls noch einer Verdünnung unterzogen werden muss. Ebenfalls nachteilig ist der große gerätetechnische Aufwand, der zum Betrieb der Kassette für die Vorbehandlung und Analyse der Probe notwendig ist, sowie der hohe Fertigungs- und Materialaufwand für die Kassette selbst. Diese Nachteile stehen einer zeitnahen, kontinuierlichen und in dem Sinne mobilen Diagnostik, bei der der Proband das Analysegerät zeitlich unbegrenzt mit sich führen kann, entgegen.

Eine andere Möglichkeit, organische Substanzen, welche in der Gasphase enthalten sind, zu bestimmen, beschreibt EP 0 634 488 A2. Hier wird ein Biosensor beschrieben, der aus einem mit Elektroden bedruckten Dickfilmsubstrat besteht, auf dem eine Feuchtigkeit absorbierende Schicht aufgebracht ist, die ein Enzym enthält, welches mit der in der Gasphase enthaltenen zu bestimmenden Substanz reagiert. Die Verwendung dieses Biosensors zur Analyse hat den Nachteil, dass das Einbringen des Sensors in die zu untersuchende Gasphase den Startpunkt der Probensammlung durch Absorption bestimmt und deren Geschwindigkeit zum einen vom Ausgangszustand des Sensors und zum anderen von der Umgebung (z.B. Feuchtigkeit und Anströmverhältnisse der Sensoroberfläche) im Gas abhängt. Deshalb ist es notwendig, eine entsprechend aufwendige Kontrolle bzw. Steuerung der Umgebungsbedingungen zu etablieren, um einen definierten Start der Messung zu gewährleisten und damit Fehlmessungen aufgrund von Artefakten während der noch nicht vollständigen Absorption der Probe aus der Gasphase zu vermeiden. Zur Konzentrationsbestimmung von Substanzen in einem relativ ungleichmäßigen Gasstrom, wie er beim Ausatmen vorliegt, ist dieses Methode ungeeignet.

Der Erfindung lag deshalb die Aufgabe zu Grunde, unter Verwendung von Sensoren ein Verfahren zur Bestimmung von Substanzen in der Ausatemluft auf der Basis von Atemkondensaten bereitzustellen, das zuverlässig reale Messwerte liefert. Aufgabe der Erfindung war es außerdem, eine den Probanden nicht belastende, preisgünstige, gegebenenfalls auch mobil verwendbare Vorrichtung zu entwickeln, die die reale Analyse der Substanzen gestattet und eine möglichst zeitnahe und kontinuierliche Diagnostik und Überwachung von Krankheiten und physiologischen Prozessen der Atmungsorgane auf der Grundlage der Konzentrationsbestimmung von für diese Prozesse relevanten Substanzen, die im Atemkondensat enthaltenen sind, gewährleistet.

Die Aufgabe der Erfindung wird mit den kennzeichnenden Merkmalen des Verfahrensanspruches 1 und des Vorrichtungsanspruches 10 gelöst. Vorteilhafte Ausgestaltungen des Verfahrens und der Vorrichtung sind Gegenstand von Unteransprüchen.

Für das erfindungsgemäße Verfahren zur Bestimmung von Substanzen in der Ausatemluft auf der Basis von Atemkondensaten gemäß Anspruch 1 werden elektrochemische Sensoren und/oder Biosensoren in Verbindung mit einem Stromversorgungs- und elektronischen Mess-, Steuer- und Auswertesystem eingesetzt. Die Abscheidung von Atemluftkondensaten erfolgt an einer funktionalisierten Kondensationsfläche, die gekühlt wird und die mit den Sensoren verbunden ist. Erfindungsgemäß erfolgt eine Funktionalisierung der Kondensationsfläche mit Stoffen, die mit Substanzen im Kondensat der Ausatemluft eine chemische Reaktion eingehen und/oder die Leitfähigkeit des Kondensates ändern. Die sich durch Kühlung bildenden Kondensattröpfchen gelangen in Abhängigkeit von den geometrischen Abmessungen der Kondensationsfläche durch Schwerkraft, Kapillarkräfte und/oder Oberflächeneffekte zu den Sensoren, wobei die durch die Substanzen erzeugten Signale bzw. Reaktionen mittels Mess-, Steuer- und Auswertesystemen bestimmt werden können. Besonders bevorzugt werden Kondensationsflächen eingesetzt, die mit einem funktionalisierten oder aktivierten organischen Polymer oder Gemischen davon beschichtet sind. Vorzugsweise handelt es sich um Schichten aus Polyvinyl, Polystyren, Polyacrylat, Polyurethan oder Cellulose-Derivaten und/oder polymeren Siliziumverbindungen. Funktionalisierte oder aktivierte Kondensationsflächen bedeutet im Sinne der Erfindung, dass sie z.B. Polymere mit Stoffen bzw. Substanzen aufweisen, welche mit Substanzen in der Ausatemluft in Wechselwirkung treten und/oder eine chemische Reaktion auslösen. So können die Polymere z.B. anorganische Alkalisalze, wie z.B. die Chloride KCl oder NaCl aufweisen, welche in dem Atemkondensat in Lösung gehen und so zu einer Veränderung (Erhöhung) der Leitfähigkeit führen und Messungen mit elektrochemischen Sensoren ermöglichen. Besonders bevorzugt werden Polymerschichten aus Polyurethan oder Polyurethangemischen, Cellulose-Derivaten und/oder polymere Siliziumverbindungen verwendet.

Die Herstellung von entsprechend funktionalisierten oder aktivierten organischen Polymeren erfolgt z.B., indem Salze oder andere Additive Polymersuspensionen oder Gemischen von Polymersuspensionen vor der Vernetzung der Polymere zugesetzt werden. Bei der Vernetzung der Polymere lagern sich die Salze oder auch andere Additive in der Polymerschicht ein. Eine andere Möglichkeit der Polymerfunktionalisierung besteht darin, chemische Reaktionspartner wie z.B. Enzyme für zu detektierende Substanzen in den Polymeren oder an deren Oberfläche zu immobilisieren. Des Weiteren können durch Verwendung bestimmter Polymere und deren chemischer oder physikalischer Oberflächenbehandlung die Benetzungseigenschaften dieser Polymere beeinflusst werden, was Auswirkungen auf die Tröpfchengröße, das Zusammenfließen der Kondensattröpfchen und somit auf den gesamten Verlauf der Probesammlung hat.

Die Oberfläche der Kondensationsflächen ist so beschaffen, dass die sich bildenden Kondensattröpfchen zu immer größeren Tröpfchen zusammenfließen. Innerhalb dieser relativ kurzen Zeitspanne nehmen sie in überraschender Weise die Stoffe (Substanzen), welche z.B. in dem Polymer enthalten sind, auf. So lässt sich wie bereits ausgeführt, z. B. die Leitfähigkeit des Kondensates beeinflussen. Des Weiteren können die zu bestimmenden Substanzen z. B. mit in der Polymerschicht immobilisierten Enzymen zu leicht detektierbaren Reaktionsprodukten reagieren. Beispielsweise kann im Atemkondensat enthaltenes Lactat durch Lactatoxidase katalysiert zu Pyruvat und Wasserstoffperoxid reagieren, welches einfach elektrochemisch detektierbar ist. Möglich ist aber auch die Funktionalisierung der Kondensationsfläche durch kovalente Anbindung von Stoffen an der Kondensationsfläche, die mit Substanzen im Kondensat der Ausatemluft eine Wechselwirkung oder eine chemische Reaktion eingehen. Beispielsweise werden Enzyme direkt an funktionalisierte polymere Oberflächen mit Hilfe von Glutardialdehyd oder an Goldoberflächen über Thiole oder an Glas oder Keramik über siliziumorganische Verbindungen kovalent angebunden.

Die Erfindung umfasst weiterhin eine Vorrichtung zur Gewinnung von Atemkondensaten und zur umgehenden Bestimmung von Substanzen, die in der Atemluft vorhanden sind, gemäß Anspruch 10. Die Vorrichtung hat dabei den großen Vorteil, dass neben normalen Standardgeräten im Klinik- und Laborbereich auch tragbare Systeme, die auch für den Point-of-Care Einsatz geeignet sind, verwendet werden können.

Nach einer bestimmten Zeit, die von der Temperatur der gekühlten funktionalisierten Fläche, von deren Oberflächeneigenschaften sowie deren geometrischen Abmessungen abhängt, gelangt das gesammelte Kondensat direkt auf die in unmittelbarer Nähe der gekühlten Fläche befindlichen Sensoren, mit denen in Verbindung mit der entsprechenden Mess- und Auswerteelektronik die im Atemkondensat enthaltenen Inhaltsstoffe bestimmt werden können. Mit entsprechenden Sensoren können Parameter des Atemkondensates bestimmt werden, wie z.B. die Wasserstoffperoxid-, die Laktat- oder die Ammoniumkonzentration.

In einer bevorzugten Variante ist mit Hilfe des Peltier-Elements in Kombination mit Temperaturfühlern, die die Umgebungstemperatur und die Temperatur der gekühlten Fläche messen, die Temperatur der gekühlten Fläche und damit der Zeitpunkt sowie die Dauer der Kondensatsammlung einfach zu steuern.

In einer weiteren bevorzugten Ausführungsvariante ist es möglich, durch Unterteilung der Kondensationsfläche in mehrere Kondensationsflächen und deren entsprechender Wärmeisolierung in Kombination mit einem oder mit mehreren Peltier-Elementen Kaskaden oder alternierende Kondensationsabläufe zu steuern. Durch die Gestaltung der Kondensationsoberflächen mit funktionalisierten Schichten kann eine planare Fluidik etabliert werden und Wechselwirkungseffekte oder chemische Prozesse können gezielt veranlasst werden. Letzteres geschieht z.B. dadurch, dass das Kondensat die Substanzen aufnimmt, die gezielt eingebracht worden sind.

Die Sensoreinheit der Vorrichtung zur Gewinnung und Analyse von Atemkondensaten besitzt eine funktionalisierte Kondensationsfläche, die auf der Basis von Polymer, Metall oder Keramik oder aus einer Kombination dieser Materialien aufgebaut ist, und einer Fläche, auf der sich die Sensoren befinden. Die die Sensoren tragenden Flächen bestehen vorzugsweise aus Keramik oder Kunststoff, bevorzugt aus Thermoplasten. Die Kondensationsfläche bzw. -flächen sind räumlich so mit dem einen oder mehrere Sensoren tragenden Element zur Sensoreinheit kombiniert, dass nach Bildung einer bestimmten Kondensatmenge diese z. B. durch Schwerkraft, Kapillarkräfte und/oder Oberflächeneffekte direkt auf den einzelnen Sensor oder die Sensoren gelangt und die Bestimmung von Inhaltsstoffen des Atemkondensates erfolgt. Form und Größe der Kondensationsschicht werden durch eine Abdeckung gestaltet und begrenzt. Diese Abdeckung kann auch so ausgestaltet sein, dass in geringem Abstand über der Oberfläche des Sensors oder der Sensoren eine zusätzliche Wand verläuft und sich so ein Raum über der Oberfläche des Sensors oder der Sensoren bildet, der durch Kapillarkräfte gefüllt werden kann. Die Sensoreinheit ist auf einem Grundelement angeordnet. Dieses kann ein Träger oder eine Halterung sein, in die die Sensoreinheit eingesteckt werden kann. Das Grundelement verfügt über entsprechende Kontaktbahnen, vorzugsweise Klemmkontakte für die Sensoren und für die Wärmeübertragung von mindestens einem ebenfalls in diesem Grundelement untergebrachten Peltier-Element und kann mit der Mess-, Auswerte- und Stromversorgungseinheit verbunden werden.

Die erfindungsgemäße Vorrichtung hat den Vorteil, dass neben der Schwerkraft durch die Ausgestaltung der erfindungsgemäßen Vorrichtung auch Kapillarkräfte und/oder Oberflächeneffekte genutzt werden können, um einerseits den Transport des Kondensates auf die Sensoren zu bewirken und/oder zu unterstützen oder andererseits das Probevolumen, welches auf die Sensoren aufgebracht wird, zu definieren. Durch die bevorzugt gewählten Klemmkontakte der Vorrichtung wird gewährleistet, dass die Sensoreinheit schnell und unkompliziert gewechselt werden kann. Der Einsatz verschiedener Konfigurationen derartiger Sensoreinheiten mit unterschiedlichen Sensoren und Polymerbeschichtungen für verschiedene Anwendungen ist dadurch möglich. In einer bevorzugten Ausgestaltung der erfmdungsgemäßen Vorrichtung ist eine Halterung mit Sensoreinheit und Peltier-Element in einer vom Probanden tragbaren Kopfhalterung ("Head-Set") untergebracht, um so eine zeitnahe und dauerhaft zumutbare Kondensatgewinnung zu gewährleisten. Als Sensoren können einzelne oder auch Kombinationen von Einweg- oder Mehrwegsensoren auf elektrochemischer aber auch auf optischer Basis oder Biosensoren je nach Erfordernis der zu bestimmenden Parameter und Substanzen zum Einsatz kommen.

### Ausführungsbeispiele

### Beispiel: Laktatbestimmung.

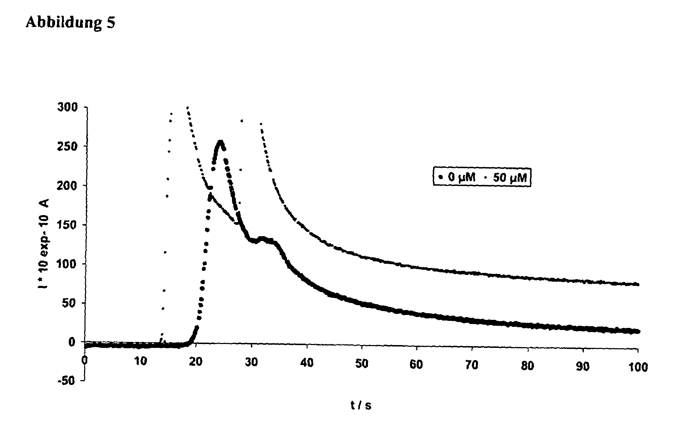

Auf einem Keramiksubstrat, das mit einer 2-Elektrodenstuktur in Dickschichtechnik versehen ist, befindet sich außerdem eine Kondensationsfläche, welche mit einer mit dem Enzym Lactatoxidase funktionalisierten Hydroxyethylcelluose-Schicht bedeckt ist. Kondensationsfläche und 2-Elektrodenstuktur auf der Keramik bilden gemeinsam die Sensoreinheit. Diese wird in eine Halterung eingesetzt, welche mit Peltier-Element, Wärmeleitbrücke und elektrischen Federkontakten ausgestattet und mit einer Mess-, Steuer- und Regeleinheit verbunden ist. Zwischen den beiden Elektroden der 2-Elektrodenstuktur wird eine Spannung von +450 mV angelegt, bei welcher das bei der vom Enzym Lactatoxidase katalysierten Reaktion mit dem im Atemkondensat vorhandenen Lactat als Nebenprodukt gebildete Wasserstofperoxid elektrochemisch oxidiert wird. Die Kondensationsfläche wird zunächst von Raumtemperatur auf 14 °C gekühlt. Danach richtet der Proband seinen Atem solange auf die Kondensationsfläche der Sensoreinheit bis sich eine bestimmte Menge Kondensat angesammelt hat und auf die Elektrodenstruktur gelangt. Das Benetzen der Sensorstruktur mit dem Atemkondensat äußert sich in einem schnellen Ansteigen und Abklingen des von der Lactatkonzentration abhängigen Sensorstroms. 30 s nach Benetzung der Elektrodenstruktur wurde für einen Lactatgehalt von 50 µmol/l im Atemkondensat ein Strom von 12 nA gemessen während bei einer laktatfreien Vergleichsprobe ebenfalls 30 s nach Benetzung ein Strom von 5 nA gemessen wurde (siehe Abbildung 5).

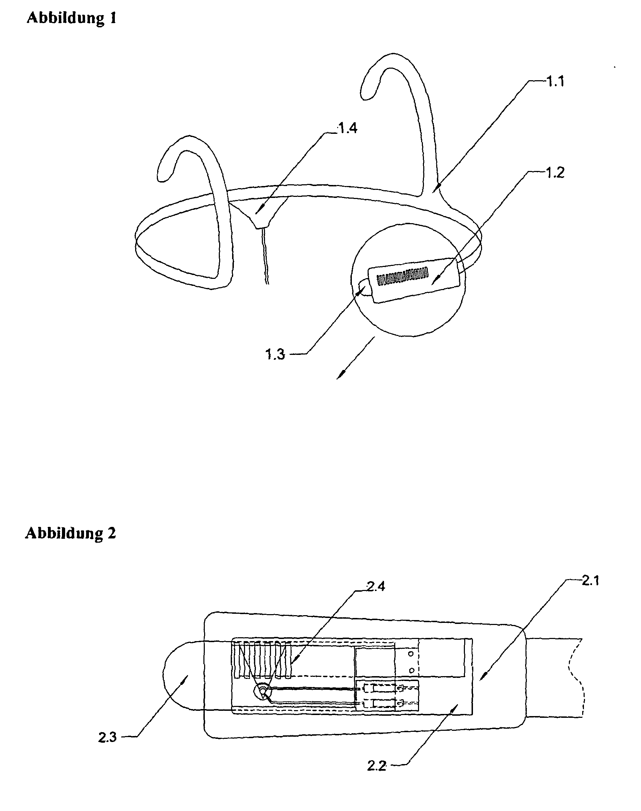

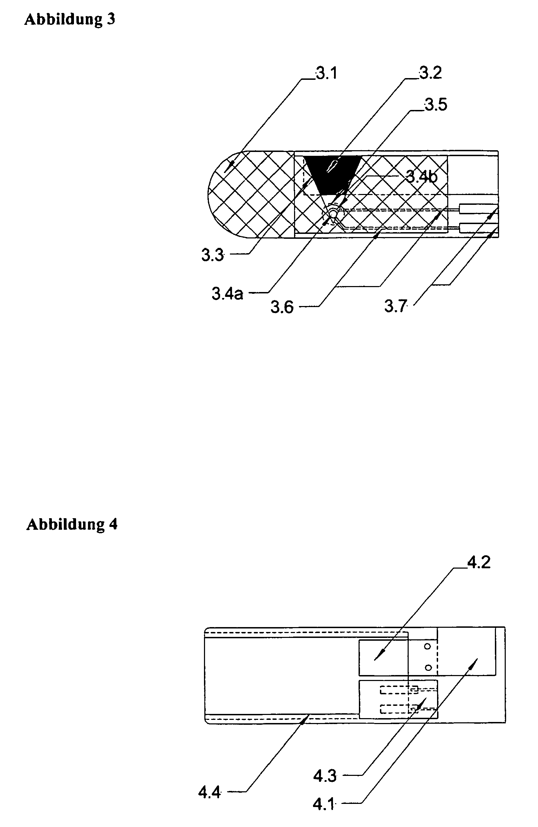

Weiterhin zeigen die Abbildungen 1 bis 4 bevorzugte Ausgestaltungen der Vorrichtung ohne dass die Erfindung darauf beschränkt werden soll.

### Abbildung 1

zeigt eine schematische Darstellung einer Kopfhalterung (1.1) mit einer vor dem Mund eines Probanden zu positionierenden erfindungsgemäßen Vorrichtung in Form einer Halterung (1.2), mit einer eingesteckten Sensoreinheit (1.3), Peltier-Element und Wärmeleitbrücke. Die in der Halterung enthaltenen Sensorkontakte und das Peltier-Element bzw. mehrere Peltier-Elemente können über entsprechende Leitungen (1.4) mit einer Mess-, Steuerungs- und Stromversorgungseinheit (ohne Abbildung), die am Körper getragen werden kann, verbunden werden.

### Abbildung_2

zeigt eine Halterung (2.2), die von einem Gehäuse (2.1) umgeben ist, welches mit Schlitzen (2.4) zum Wärme- und Stoffaustausch mit der Umgebung versehen ist, mit einer in diese Halterung eingesteckten Sensoreinheit (2.3), Peltier-Element und Wärmeleitbrücke.

### Abbildung 3

zeigt schematisch eine Sensoreinheit mit einem Griffstück (3.1), einer Abdeckung (Schraffur), welche mit einem Ausschnitt (3.3) versehen ist, der eine einheitliche Kondensationsfläche (3.2), sowie die Reaktionsfläche einer einzelnen schematisch dargestellten Biosensorstruktur (3.5) freilässt. Der Biosensor besteht beispielsweise aus Elektrodenstrukturen, der Arbeitselektrode (3.4a), einer Gegen-/Referenzelektrode (3.4b), Ableit- (3.6) und Kontaktstrukturen (3.7), die auf eine Wärme isolierende Schicht aufgedruckt sind. Die Elektrodenstruktur ist z.B. mit einer Enzym-Polymer-Schicht beschichtet.

### Abbildung 4

zeigt schematisch eine Halterung mit einem Peltier-Element (4.1), einen Kontaktblock für eine Biosensorstruktur (4.3), eine Wärmeleitbrücke (4.2) und Einschubnuten (4.4) für das Sensorelement.

### Abbildung 5

zeigt die Abhängigkeit des Stromsignals von der Lactat - Konzentration im Atemkondensat bei einem Ausführungsbeispiel

## Patentansprüche

1. Verfahren zur Gewinnung von Atemkondensaten und zur sofortigen Bestimmung ihrer Inhaltsstoffe, **dadurch gekennzeichnet, dass** die Ausatemluft an mindestens einer funktionalisierten oder aktivierten Fläche (3.2) unter Kühlung kondensiert wird, die gebildeten Kondensattröpfchen direkt zu einem elektrochemischen Sensor und/oder Biosensor (3.5) geleitet werden, die dort erzeugten Signale bzw. Reaktionen mittels eines mit dem Sensor (3.5) in Verbindung stehenden Stromversorgungs- und Mess-, Steuer- und Auswertesystems ermittelt und die Inhaltsstoffe bestimmt werden, wobei die Kondensattröpfchen in Abhängigkeit von der geometrischen Anordnung und Abmessung der Fläche (3.2) durch Schwerkraft, Kapillarkräfte und/oder Oberflächeneffekte zu dem Sensor (3.5) gelangen, und als funktionalisierende oder aktivierende Stoffe Substanzen eingesetzt werden, welche die Leitfähigkeit des Kondensates verändern und/oder Stoffe, welche mit den zu untersuchenden Substanzen chemisch reagieren.

2. Verfahren nach Anspruch 1, **dadurch gekennzeichnet, dass** die Kondensationsfläche (3.2) eine Polymerschicht umfasst, die mit Stoffen funktionalisiert oder aktiviert ist, die mit dem Kondensat und/oder mit enthaltenen zu analysierenden Inhaltsstoffen eine Wechselwirkung und/oder eine chemische Reaktion eingehen.

3. Verfahren nach Anspruch 1 oder 2, **dadurch gekennzeichnet, dass** die funktionalisierte oder aktivierte Fläche (3.2) eine organische oder anorganische Polymerschicht oder Gemische umfasst, vorzugsweise Polyvinyl, Polystyrol, Polyacrylat, Polyurethan, Cellulose-Derivate und/oder polymere Siliziumverbindungen.

4. Verfahren nach Anspruch 1, **dadurch gekennzeichnet, dass** Alkalisalze und/oder Enzyme zur Funktionalisierung eingesetzt werden.

5. Verfahren nach Anspruch 1, **dadurch gekennzeichnet, dass** die Kondensationsfläche (3.2) durch kovalente Anbindung von Enzymen funktionalisiert ist.

6. Verfahren nach einem der Ansprüche 1 bis 5, **dadurch gekennzeichnet, dass** die Menge des Kondensates über die Zeit und/oder die Kühltemperatur gesteuert wird.

7. Verfahren nach einem der Ansprüche 1 bis 6, **dadurch gekennzeichnet, dass** mehrere Kondensationsflächen (3.2), die gleichartig und/oder unterschiedlich funktionalisiert sind, verwendet werden, und die Kondensat-Inhaltsstoffe über Kaskaden oder alternierend unter Verwendung gleich- oder verschiedenartiger Sensoren (3.5) in Abhängigkeit der zu bestimmenden Substanzen bestimmt werden.

8. Verfahren nach Anspruch 7, **dadurch gekennzeichnet, dass** parallel unterschiedliche Substanzen bestimmt werden, wobei die funktionalisierten Kondensationsflächen (3.2) und/oder die Sensoren (3.5) unterschiedlich ausgestattet sind.

9. Verfahren nach einem der Ansprüche 1 bis 8, **dadurch gekennzeichnet, dass** durch Messung der Umgebungstemperatur die Kühltemperatur, der Zeitpunkt und die Dauer der Kondensatgewinnung gesteuert werden.

10. Vorrichtung zur Gewinnung von Atemkondensaten und zur umgehenden Bestimmung ihrer Inhaltsstoffe umfassend
- ein Grundelement in Form eines Trägers oder einer Halterung (2.2), die eine Sensoreinheit (2.3), mindestens ein Peltier-Element (4.1) zur Kühlung sowie eine verbindende Wärmeleitbrücke (4.2) umfasst, wobei die Sensoreinheit (2.3) mindestens eine Kondensationsfläche (3.2), die mit Substanzen, die die Leitfähigkeit des Kondensates verändern und/oder Stoffen, die mit den zu untersuchenden Substanzen chemisch reagieren, funktionalisiert oder aktiviert ist, sowie mindestens einen elektrochemischen Sensor und/oder Biosensor (3.5) aufweist,
wobei die funktionalisierte bzw. aktivierte Kondensationsfläche (3.2) und Sensor (3.5) oder Sensoren so verbunden sind, dass ein Kondensat unmittelbar und direkt in Abhängigkeit von der geometrischen Anordnung und Abmessung der Kondensationsfläche (3.2) durch Schwerkraft, Kapillarkräfte und/oder Oberflächeneffekte zum Sensor (3.5) fließen kann,
- sowie ein Stromversorgungs- und elektronisches Mess-, Steuer- und Auswertesystem.

11. Vorrichtung nach Anspruch 10, **dadurch gekennzeichnet, dass** die Kondensationsfläche (3.2) durch kovalente Anbindung von Enzymen funktionalisiert ist.

12. Vorrichtung nach Anspruch 11, **dadurch gekennzeichnet, dass** die Kondensationsfläche (3.2) mit organischen und/oder anorganischen Polymeren beschichtet ist, die mit Stoffen funktionalisiert oder aktiviert sind, die mit den zu analysierenden Inhaltsstoffen in der Atemluft Wechselwirkungen und/oder chemische Reaktionen eingehen.

13. Vorrichtung nach Anspruch 10 bis 12, **dadurch gekennzeichnet, dass** sie einen Temperaturfühler umfasst, der die Umgebungstemperatur misst.

14. Vorrichtung nach einem der Ansprüche 10 bis 13, **dadurch gekennzeichnet, dass** mehrere Kondensationsflächen (3.2), welche über Wärmeleitbrücken (4.2) mit einem oder mehreren Peltier-Elementen (4.1) verbunden sind, und zugehörige Sensoren (3.5) vorhanden sind, welche untereinander isoliert angeordnet sind, wobei die Kondensationsflächen (3.2) eine gleichartige und/oder unterschiedliche Funktionalisierung aufweisen und die Sensoren (3.5) gleichartig und/oder verschieden sind.

15. Vorrichtung nach einem der Ansprüche 10 bis 14, **dadurch gekennzeichnet, dass** die funktionalisierte Kondensationsfläche (3.2) auf der Basis von Polymeren, Keramik, Metall oder aus einer Kombination aus diesen Materialien aufgebaut ist.

16. Vorrichtung nach einem der Ansprüche 10 bis 15, **dadurch gekennzeichnet, dass** die Kondensationsfläche (3.2) mit Polymeren, vorzugsweise Polyvinylpolymere, Polystyrene, Polyacrylate, Polyurethane, Cellulose-Derivate und/oder polymere Siliziumverbindungen und/oder Polymermischungen beschichtet sind, welche mit Alkalisalzen und/oder Enzymen funktionalisiert sind.

17. Vorrichtung nach Anspruch 16, **dadurch gekennzeichnet, dass** die Polymerbeschichtung mit Alkalichloriden funktionalisiert ist.

18. Vorrichtung nach Anspruch 17, **dadurch gekennzeichnet, dass** die Polymerbeschichtung mit Enzymen funktionalisiert ist.

19. Vorrichtung nach Anspruch 17, **dadurch gekennzeichnet, dass** die Polymerbeschichtung mit anorganischen Salzen funktionalisiert ist.

20. Vorrichtung nach einem der Ansprüche 10 bis 19, **dadurch gekennzeichnet, dass** die Funktionalisierung der Kondensationsfläche (3.2) durch kovalente Anbindung von Enzymen an die Kondensationsfläche (3.2) erfolgt.

21. Vorrichtung nach einem der Ansprüche 10 bis 20, **dadurch gekennzeichnet, dass** die den Sensor (3.5) tragende Fläche aus Keramik oder einem Kunststoff besteht, vorzugsweise aus Thermoplasten.

22. Vorrichtung nach einem der Ansprüche 10 bis 21, **dadurch gekennzeichnet, dass** die jeweilige Kondensationsfläche (3.2) definierte geometrische Abmessungen besitzt.

23. Vorrichtung nach einem der Ansprüche 10 bis 22, **dadurch gekennzeichnet, dass** der Sensor (3.5) eine Abdeckung in der Art aufweist, dass ein Raum entsteht, wodurch das Kondensat durch Kapillarkräfte auf den Sensor (3.5) fließt.

24. Vorrichtung nach einem der Ansprüche 10 bis 23, **dadurch gekennzeichnet, dass** sich das Grundelement mit Sensoreinheit (2.3), Peltier-Element (4.1) und Wärmeleitbrücke (4.2) in einem Gehäuse (2.1) befindet, das mit Schlitzen oder einem Gitter versehen ist.

25. Vorrichtung nach einem der Ansprüche 10 bis 24, **dadurch gekennzeichnet, dass** sie eine Kopfhalterung (1.1) einschließt und so konstruiert ist, dass die Kondensationsfläche (3.2) direkt vor dem Mund eines Probanden positioniert werden kann.

26. Vorrichtung nach einem der Ansprüche 10 bis 25, **dadurch gekennzeichnet, dass** sie mit einem tragbaren Stromversorgungs- und Auswertesystem verbunden ist.

27. Verwendung von funktionalisierten oder aktivierten Kondensationsflächen (3.2) für die Abscheidung von Kondensat aus der Ausatemluft zur sofortigen, unmittelbaren Bestimmung von deren Inhaltsstoffen mittels an sich bekannter elektrochemischer Analyseeinrichtungen in einem Verfahren gemäß einem der Ansprüche 1 bis 9.

## Claims

1. Method for collecting respiratory condensates and immediately determining their constituents, **characterized in that** the exhaled air is condensed on at least one functionalized or activated surface (3.2) with cooling; the condensate droplets thereby formed are sent directly to an electrochemical sensor and/or biosensor (3.5), the signals and/or reactions generated there are detected by means of a power supply system and a measurement, control and analysis system connected to the sensor (3.5) and the constituents are determined, the condensate droplets reaching the sensor (3.5) as a result of gravitational force, capillary forces and/or surface effects, depending on the geometric arrangement and dimensions of the surface (3.2), and substances that alter the conductivity of the condensate and/or substances that react chemically with the substances to be analyzed are used as the functionalizing or activating substances.

2. Method according to Claim 1, **characterized in that** the condensation surface (3.2) comprises a polymer layer which is functionalized or activated with substances that enter into an interaction and/or a chemical reaction with the condensate and/or with constituents that are to be analyzed and are contained therein.

3. Method according to Claim 1 or 2, **characterized in that** the functionalized or activated surface (3.2) comprises an organic or inorganic polymer layer of mixtures, preferably polyvinyl, polystyrene, polyacrylate, polyurethane, cellulose derivatives and/or silicon polymer compounds.

4. Method according to Claim 1, **characterized in that** alkali salts and/or enzymes are used for functionalization.

5. Method according to Claim 1, **characterized in that** the condensation surface (3.2) is functionalized by covalent bonding of enzymes.

6. Method according to any one of Claims 1 through 5, **characterized in that** the amount of condensate is controlled as a function of time and/or the cooling temperature.

7. Method according to any one of Claims 1 through 6, **characterized in that** multiple condensation surfaces (3.2) which are functionalized similarly and/or differently are used, and the condensate constituents are determined via cascades or by using similar or different sensors (3.5) as a function of the substances to be determined.

8. Method according to Claim 7, **characterized in that** different substances are determined in parallel, the functionalized condensation surfaces (3.2) and/or sensors (3.5) being equipped differently.

9. Method according to any one of Claims 1 through 8, **characterized in that** the cooling temperature, the point in time and duration of condensate collection are controlled by measuring the ambient temperature.

10. Device for collecting respiratory condensates and for immediately determining their constituents, comprising
- a base element in the form of carrier or a holder (2.2), comprising a sensor unit (2.3), at least one Peltier element (4.1) for cooling and a connecting heat conducting bridge (4.2), whereby the sensor unit (2.3) has at least one condensation surface (3.2) which is functionalized or activated with substances that alter the conductivity of the condensate and/or substances that react chemically with the substances to be analyzed, and comprising at least one electrochemical sensor and/or biosensor (3.5), whereby the functionalized and/or activated condensation surface (3.2) and sensor (3.5) or sensors are connected in such a way that the condensate can flow directly to the sensor (3.5) through gravitational force, capillary forces and/or surface effects, depending on the geometric arrangement and dimensions of the condensation surface (3.2),
- as well as a power supply system and an electronic measurement, control and analysis system.

11. Device according to Claim 10, **characterized in that** the condensation surface (3.2) is functionalized by covalent bonding of enzymes.

12. Device according to Claim 11, **characterized in that** the condensation surface (3.2) is coated with organic and/or inorganic polymers which are functionalized or activated with substances that enter into interactions and/or chemical reactions with the constituents in the respiratory air to be analyzed.

13. Device according to Claims 10 through 12, **characterized in that** it comprises a temperature sensor that measures the ambient temperature.

14. Device according to any one of Claims 10 through 13, **characterized in that** several condensation surfaces (3.2) which are connected to one or more Peltier elements (4.1) via heat conducting bridges (4.2) and the respective sensors (3.5) are present, arranged so they are insulated from one another, the condensation surfaces (3.2) having a similar and/or different functionalization and the sensors (3.5) being similar and/or different.

15. Device according to any one of Claims 10 through 14, **characterized in that** the functionalized condensation surface (3.2) is constructed based on polymers, ceramics, metals or a combination of these materials.

16. Device according to any one of Claims 10 through 15, **characterized in that** the condensation surface (3.2) is coated with polymers, preferably polyvinyl polymers, polystyrenes, polyacrylates, polyurethanes, cellulose derivatives and/or silicon polymer compounds and/or polymer blends which are functionalized with alkali salts and/or enzymes.

17. Device according to Claim 16, **characterized in that** the polymer coating is functionalized with alkali chlorides.

18. Device according to Claim 17, **characterized in that** the polymer coating is functionalized with enzymes.

19. Device according to Claim 17, **characterized in that** the polymer coating is functionalized with inorganic salts.

20. Device according to any one of Claims 10 through 19, **characterized in that** functionalization of the condensation surface (3.2) is achieved by covalent bonding of enzymes to the condensation surface (3.2).

21. Device according to any one of Claims 10 through 20, **characterized in that** the surface carrying the sensor (3.5) is made of ceramic or a plastic, preferably a thermoplastic.

22. Device according to any one of Claims 10 through 21, **characterized in that** the respective condensation surface (3.2) has defined geometric dimensions.

23. Device according to any one of Claims 10 through 22, **characterized in that** the sensor (3.5) has a cover of a type such that it forms a space through which the condensate flows to the sensor (3.5) through capillary forces.

24. Device according to any one of Claims 10 through 23, **characterized in that** the base element with the sensor unit (2.3), Peltier element (4.1) and heat conducting bridge (4.2) is provided in a housing (2.1) equipped with slots or a grating.

25. Device according to any one of Claims 10 through 24, **characterized in that** it includes a headset (1.1) and is designed so that the condensation surface (3.2) can be positioned directly in front of the mouth of a test subject.

26. Device according to any one of Claims 10 through 25, **characterized in that** it is connected to a portable power supply system and analysis system.

27. Use of functionalized or activated condensation surfaces (3.2) for deposition of condensate from the exhaled air for immediate and direct determination of the constituents thereof by means of essentially known electrochemical analysis equipment in a method according any one of claims 1 to 9.

## Revendications

1. Procédé d'obtention de condensats de respiration et de détermination immédiate de leurs constituants, **caractérisé en ce que** l'air expiré est condensé sur au moins une surface fonctionnalisée ou activée (3.2) sous un refroidissement, les gouttelettes de condensat formées sont amenées directement vers un capteur électrochimique et/ou un biocapteur (3.5), les signaux ou les réactions y étant produites sont déterminés au moyen d'un système d'alimentation électrique, de mesure, de commande et d'évaluation en liaison avec le capteur (3.5) et les constituants sont déterminés, moyennant quoi les gouttelettes de condensat parviennent au capteur (3.5) selon la disposition géométrique et les dimensions de la surface (3.2), par force de gravité, forces de capillarité et/ou effets de surface, et on utilise, en tant que substances fonctionnalisantes ou activantes, des substances qui modifient la conductivité du condensat et/ou des substances qui réagissent chimiquement avec les substances à analyser.

2. Procédé selon la revendication 1, **caractérisé en ce que** la surface de condensation (3.2) comprend une couche de polymère, qui est fonctionnalisée ou activée avec des substances qui interviennent dans une interaction et/ou une réaction chimique avec le condensat et/ou les constituants contenus à analyser.

3. Procédé selon la revendication 1 ou 2, **caractérisé en ce que** la surface fonctionnalisée ou activée (3.2) comprend une couche de polymère organique ou minéral ou des mélanges, de préférence de polyvinyle, polystyrène polyacrylate, polyuréthane, dérivés de cellulose, et/ou de composés de silicium polymères.

4. Procédé selon la revendication 1, **caractérisé en ce qu'**on utilise des sels alcalins et/ou des enzymes pour la fonctionnalisation.

5. Procédé selon la revendication 1, **caractérisé en ce que** la surface de condensation (3.2) est fonctionnalisée par une liaison covalente d'enzymes.

6. Procédé selon l'une des revendications 1 à 5, **caractérisé en ce que** la quantité de condensat est commandée par le temps et/ou la température de refroidissement.

7. Procédé selon l'une des revendications 1 à 6, **caractérisé en ce qu'**on utilise plusieurs surfaces de condensation (3.2), qui sont fonctionnalisées de la même manière et/ou différemment et les constituants du condensat sont déterminés en cascade ou alternativement en utilisant des capteurs (3.5) de type similaire ou différent en fonction des substances à déterminer.

8. Procédé selon la revendication 7, **caractérisé en ce que** les substances sont déterminées en parallèle, moyennant quoi les surfaces de condensation (3.2) fonctionnalisées et/ou les capteurs (3.5) sont équipées différemment.

9. Procédé selon l'une des revendications 1 à 8, **caractérisé en ce que** la température de refroidissement, le moment et la durée de l'obtention de condensat sont commandés en mesurant la température environnante.

10. Dispositif d'obtention de condensats de respiration et de détermination immédiate de leurs constituants, comprenant :
- un élément de base sous forme d'un support ou d'une fixation (2.2), qui comprend un organe de capteur (2.3), au moins un élément Peltier (4.1) de refroidissement ainsi qu'un pont thermoconducteur de jonction (4.2), moyennant quoi l'organe de capteur (2.3) comprend au moins une surface de condensation (3.2), qui est fonctionnalisée ou activée avec des substances qui modifient la conductivité du condensat et/ou des substances qui réagissent chimiquement avec les substances à analyser, et au moins un capteur électrochimique et/ou un biocapteur (3.5), moyennant quoi la surface de condensation fonctionnalisée ou activée (3.2) et le capteur (3.5) ou les capteurs sont reliées de telle manière qu'un condensat peut s'écouler immédiatement et directement en fonction de la disposition géométrique et des dimensions de la surface de condensation (3.2) par force de gravité, forces capillaires et/ou effets de surface vers le capteur (3.5),
- et un système d'alimentation électrique et un système de mesure, de commande et d'évaluation électronique.

11. Dispositif selon la revendication 10, **caractérisé en ce que** la surface de condensation (3.2) est fonctionnalisée par liaison covalente des enzymes.

12. Dispositif selon la revendication 11, **caractérisé en ce que** la surface de condensation (3.2) est revêtue de polymères organiques et/ou minéraux, qui sont fonctionnalisés ou activés avec des substances, qui interviennent dans des interactions et/ou des réactions chimiques avec les constituants à analyser dans l'air expiré.

13. Dispositif selon l'une des revendications 10 à 12, **caractérisé en ce qu'**il comprend une sonde de température, qui mesure la température environnante.

14. Dispositif selon l'une des revendications 10 à 13, **caractérisé en ce qu'**il y a plusieurs surfaces de condensation (3.2), qui sont reliées par un pont thermoconducteur (4.2) avec un ou plusieurs éléments Peltier (4.1), et des capteurs (3.5) correspondantes, qui sont disposées isolément l'une par rapport à l'autre, moyennant quoi les surfaces de condensation (3.2) comprennent une fonctionnalisation semblable et/ou différente et les capteurs (3.5) sont similaires ou différentes.

15. Dispositif selon l'une des revendications 10 à 14, **caractérisé en ce que** la surface de condensation (3.2) fonctionnalisée est constituée à base de polymères, céramique, métal ou d'une combinaison de ces matériaux.

16. Dispositif selon l'une des revendications 10 à 15, **caractérisé en ce que** les surfaces de condensation (3.2) sont revêtues avec des polymères, de préférence avec des polymères de polyvinyle, de polystyrène, polyacrylate, polyuréthane, dérivés de cellulose et/ou de composés de silicium polymères et/ou de mélanges de polymères, qui sont fonctionnalisés avec des sels alcalins et/ou des enzymes.

17. Dispositif selon la revendication 16, **caractérisé en ce que** le revêtement de polymère est fonctionnalisé avec des chlorures d'alcalin.

18. Dispositif selon la revendication 17, **caractérisé en ce que** le revêtement de polymère est fonctionnalisé avec des enzymes.

19. Dispositif selon la revendication 17, **caractérisé en ce que** le revêtement de polymère est fonctionnalisé avec des sels minéraux.

20. Dispositif selon l'une des revendications 10 à 19, **caractérisé en ce que** la fonctionnalisation de la surface de condensation (3.2) s'effectue par liaison covalente d'enzymes à la surface de condensation (3.2).

21. Dispositif selon l'une des revendications 10 à 20, **caractérisé en ce que** la surface portant le capteur (3.5) est constituée de céramique ou d'une matière plastique, de préférence de matière thermoplastique.

22. Dispositif selon l'une des revendications 10 à 21, **caractérisé en ce que** la surface de condensation (3.2) respective possède des dimensions géométriques définies.

23. Dispositif selon l'une des revendications 10 à 22, **caractérisé en ce que** le capteur (3.5) comprend un recouvrement de telle sorte qu'il se forme un espace au travers duquel le condensat s'écoule par des forces de capillarité sur le capteur (3.5).

24. Dispositif selon l'une des revendications 10 à 23, **caractérisé en ce que** l'élément de base se trouve avec l'organe de capteur (2.3), l'élément Peltier (4.1) et le pont thermoconducteur (4.2) dans un boîtier (2.1), qui est pourvu de rainures ou d'un quadrillage.

25. Dispositif selon l'une des revendications 10 à 24, **caractérisé en ce qu'**il inclut une fixation sur la tête (1.1) et il est construit de telle sorte que la surface de condensation (3.2) peut être positionnée directement devant la bouche d'un sujet.

26. Dispositif selon l'une des revendications 10 à 25, **caractérisé en ce qu'**il est relié avec un système d'alimentation électrique et d'évaluation portable.

27. Utilisation de surfaces de condensation fonctionnalisées ou activées (3.2) pour la précipitation de condensat de l'air expiré pour une détermination immédiate, directe de ses constituants au moyen de dispositifs électrochimiques d'analyse connus dans un procédé selon l'une des revendications 1 à 9.
